# EUROPÄISCHE PATENTSCHRIFT

(11) **EP 3 454 941 B1**
(45) Veröffentlichungstag und Bekanntmachung des Hinweises auf die Patenterteilung: **24.02.2021**
(21) Anmeldenummer: 17722748.5
(22) Anmeldetag: 09.05.2017
(51) Int. Cl.: A61N 2/02

(54) **MOBILE BEHANDLUNGSVORRICHTUNG ZUR BEHANDLUNG MIT KERNSPINRESONANZEN**
MOBILE TREATMENT DEVICE FOR THE TREATMENT USING NUCLEAR SPIN RESONANCES
DISPOSITIF DE TRAITEMENT MOBILE PERMETTANT UN TRAITEMENT PAR RÉSONANCE MAGNÉTIQUE NUCLÉAIRE

(30) Priorität: 10.05.2016 DE 102016108599
(43) Veröffentlichungstag der Anmeldung: 20.03.2019
(73) Patentinhaber: Muntermann, Axel, 35580 Wetzlar-Nauborn (DE)
(72) Erfinder: Muntermann, Axel, 35580 Wetzlar-Nauborn (DE)
(74) Vertreter: Blumbach · Zinngrebe Patentanwälte PartG mbB
(86) Internationale Anmeldenummer: PCT/EP2017/061036
(87) Internationale Veröffentlichungsnummer: WO 2017/194529

(56) Entgegenhaltungen:
- EP-A1- 1 741 467
- WO-A1-99/46157
- WO-A1-99/55420
- WO-A2-2009/156117
- FR-A1- 2 290 224
- Anonymous: "Height-adjustable over chair table for healthcare facilities (on casters) OC/1 series - 进口采购", , 14. Dezember 2014 (2014-12-14), XP055390938, Gefunden im Internet: URL:http://www.bpress.cn/im/bristol-maid-h ospital-metalcraft-height-adjustable-over- chair-table-for-healthcare-facilities-on-c asters-oc-1-series-4358/ [gefunden am 2017-07-14]
- Anonymous: "Over-bed table / on casters / height-adjustable EBT005 - 进口采购", , 14. Dezember 2014 (2014-12-14), XP055390939, Gefunden im Internet: URL:http://www.bpress.cn/im/bristol-maid-h ospital-metalcraft-over-bed-table-on-caste rs-height-adjustable-ebt005-4356/ [gefunden am 2017-07-14]
- Better Enterprise: "Product", , 1. Januar 2015 (2015-01-01), XP055390909, Gefunden im Internet: URL:http://www.bettercarts.com/products.as p [gefunden am 2017-07-14]

## Beschreibung

### Gebiet der Erfindung

Die Erfindung betrifft eine mobil ausgestaltete Behandlungsvorrichtung, mittels welcher sich im Gewebe eines Benutzers Kernspinresonanzen erzeugen lassen. Die mobile Behandlungsvorrichtung ist zur Behandlung von Gliedmaßen ausgebildet.

### Hintergrund der Erfindung

Vorrichtungen zur Behandlung eines Benutzers mit Kernspinresonanzen sind bekannt. Diese können sowohl zu Therapiezwecken als auch zu kosmetischen Zwecken, etwa zur Behandlung von Cellulite, verwendet werden.

Eine Vorrichtung zur Behandlung mit Kernspinresonanzen ist beispielsweise aus der Patentschrift DE 10 2008 029 415 B4 (Axel Muntermann) bekannt.

Es handelt sich dabei im Konkreten um einen U-förmig ausgebildeten Applikator, in welchem Spulen angeordnet sind, über die im zu behandelnden Gewebe eine Kernspinresonanz erzielt werden kann.

In einer Ausführungsvariante ist eine Liege relativ zu dem Applikator verschiebbar, um verschiedene Körperzonen behandeln zu können. In der Praxis sind derartige Vorrichtungen in der Regel als stationär aufgestellte Geräte angeordnet, auf welche sich der Patient setzen oder legen muss. Weiter aus der Praxis bekannt sind ähnlich aufgebaute Vorrichtungen, welche lediglich kleiner sind und welche der Behandlung von Gliedmaßen dienen.

Nachteilig ist dabei, dass die Vorrichtung stationär angeordnet ist und dass der Patient entsprechend positioniert werden muss, dass die zu behandelnden Gliedmaßen sich in der Behandlungszone befinden.

Dies kann unbequem und bei Patienten mit Bewegungseinschränkungen unter Umständen auch gar nicht möglich sein.

Das Dokument EP 1 741 467 A1 zeigt eine Vorrichtung zur Behandlung mit elektromagnetischen Feldern, bei welcher eine Gliedmaße auf eine höhenverstellbare Auflage platziert werden kann. Das Dokument FR 2 290 224 A1 zeigt eine Vorrichtung zur Behandlung mit magnetischen Feldern.

Höhenverstellbare Tische, insbesondere für den medizinischen Bereich, sind aus folgenden Veröffentlichungen bekannt:
- Height-adjustable over chair for healthcare facilities (on casters) OC/1 series",
   Gefunden im Internet:
   URL:http://www.bpress.cn/im/bristol-maid-hospitalmetalcraft-height-adjustable-over-chair-table-for healthcare-facilities-on-casters-oc-1-series-4358/
- "Over-bed table / on casters / height-adjustable table EBT005",
   Gefunden im Internet:
   URL:http://www.bpress.cn/im/bristol-maid-hospitalmetalcraft-over-bed-table-on-casters-height-adjustable-ebt005-4356/
- Better Enterprise: "Products",
   Gefunden im Internet:
   URL:http://www.bettercarts.com/products.asp.

Das Dokument WO 99/46157 A1 zeigt einen Wagen mit einer automatischen Bremse, die durch Drücken eines Handgriffs, mit welchem der Wagen geschoben wird, deaktiviert wird.

Das Dokument WO 2009/156 117 A2 zeigt eine Vorrichtung zur Magnetfeldtherapie.

### Aufgabe der Erfindung

Der Erfindung liegt demgegenüber die Aufgabe zugrunde, eine Behandlungsvorrichtung bereitzustellen, mit welcher sich Kernspinresonanzen im zu behandelnden Gewebe erzeugen lassen und welche sich in flexibler Weise an die Anatomie unterschiedlicher Gliedmaßen und/oder an die Anatomie unterschiedlicher Benutzer anpassen lässt.

### Zusammenfassung der Erfindung

Die Aufgabe der Erfindung wird bereits durch eine mobile Behandlungsvorrichtung mit einer Auflage für die Gliedmaßen eines Benutzers nach Anspruch 1 gelöst.

Bevorzugte Ausführungsformen und Weiterbildungen der Erfindung sind dem Gegenstand der abhängigen Ansprüche, der Beschreibung sowie den Figuren zu entnehmen.

Die Erfindung betrifft eine mobile Behandlungsvorrichtung. Unter einer mobilen Behandlungsvorrichtung wird eine Vorrichtung verstanden, welche von einer Person bewegt werden kann und welche insbesondere Räder umfasst, um zum Benutzer geschoben werden zu können.

Die Behandlungsvorrichtung umfasst eine Auflage für die Gliedmaßen eines Benutzers. Die Auflage ist insbesondere als Platte ausgebildet und so dimensioniert, dass sie die Gliedmaßen eines Benutzers, also insbesondere Arm, Hand, Ellenbogen, Bein, Knie oder Fuß aufnehmen kann.

In einem über der Auflage angeordneten Behandlungsvolumen sind Kernspinresonanzen im Gewebe des Benutzers erzeugbar.

Vorzugsweise umfasst die Behandlungsvorrichtung hierfür Seitenteile, in denen jeweils eine Spule angeordnet ist. Diese Spulen stehen in Helmholtz-Konfiguration und mittels der Spulen kann ein homogenes Magnetfeld im Behandlungsvolumen erzeugt werden.

Senkrecht zu diesem Magnetfeld wird ein magnetisches Wechselfeld eingestrahlt, vorzugsweise mittels einer Spule, welche in oder unter der Auflage angeordnet ist.

Bei entsprechend abgestimmten Feldern kann eine Kernspinresonanz erzielt werden. Vorzugsweise wird die Feldstärke des von den Spulen in den Seitenteilen angeordneten Magnetfeldes periodisch verändert (gesweept), um periodisch die Resonanzbedingung für die Erzielung von Kernspinresonanzen zu erreichen.

Gemäß der Erfindung ist die Auflage höhenverstellbar.

Das mobile Gerät kann so zum Benutzer gefahren und auf die Höhe der jeweiligen Gliedmaße eingestellt werden.

Vorzugsweise ist die Höhe um zumindest 50 cm veränderbar. So lassen sich sowohl die unteren als auch die oberen Gliedmaßen eines Benutzers auf die Auflage legen.

Die Höhenverstellung erfolgt vorzugsweise über einen Hubmechanismus, der mittels eines Pedals betätigbar ist. So kann der Bediener der Behandlungsvorrichtung mit dem Fuß die Höhe der Auflage verändern und hat dabei gleichzeitig die Hände frei, etwa um die Gliedmaßen des Patienten zunächst festzuhalten oder um die Behandlungsvorrichtung zu bedienen.

Der Hubmechanismus wird insbesondere pneumatisch betätigt, beispielsweise mittels einer Gasdruckfeder.

Bei einer Weiterbildung der Erfindung ist die Auflage in einer horizontal verlaufenden Achse drehbar ausgebildet. Die Auflage kann so nicht nur in der Höhe verstellt werden, sondern der Winkel der Auflage kann gegenüber der Horizontalen variiert werden. Insbesondere ist vorgesehen, dass gegenüber einer horizontalen Ausrichtung die Auflage um zumindest 10° verkippt werden kann.

So kann die Auflage sich gleichzeitig einer schräg stehenden Gliedmaße anpassen.

Bei einer Weiterbildung der Erfindung umfasst die Behandlungsvorrichtung einen Rastmechanismus, um die Auflage in verschiedenen Winkeln zu fixieren.

Die mobile Behandlungsvorrichtung ein Gestell mit Rädern aufweist.

Gemäß der Erfindung ist das Gestell U-förmig ausgebildet, wobei das U-förmige ausgebildete Gestell die Räder aufweist und das horizontale Grundgerüst der Behandlungsvorrichtung bildet.

Es erstreckt sich, ausgehend von dem Gestell, an einer Seite des durch das Gestell gebildeten U, eine Säule in vertikaler Richtung, wobei die Auflage an der Säule und über dem Gestell angeordnet ist.

Die Auflage kann so entlang der Säule über dem Gestell hoch- und runtergefahren werden.

Insbesondere ist vorgesehen, dass die Hubmechanik in die Säule integriert ist.

Ein U-förmiges Gestell, an dessen Ecken die Räder angeordnet sind, zieht eine hohe Standsicherheit nach sich. Gleichzeitig hat es den Vorteil, dass die offene Seite des U ein nahes Heranfahren an den Benutzer ermöglicht.

Gemäß der Erfindung ist die Säule mit der Auflage an einer Seite des U angeordnet, welche kürzer ausgebildet ist als die gegenüberliegende Seite, insbesondere, welche zumindest 20 % kürzer ist.

Dies ermöglicht ebenfalls ein nahes Heranfahren an den Benutzer, beispielsweise an die Kante eines Bettes oder eines Stuhls, auf dem der Benutzer sitzt und gleichzeitig eine hohe Standsicherheit.

Bei einer Weiterbildung der Erfindung weist die mobile Behandlungsvorrichtung einen Griff zum Schieben der Behandlungsvorrichtung auf.

Dieser ist insbesondere in etwa auf Hüfthöhe angeordnet.

Bei einer Ausführungsform ist der Griff mit einer Bremse gekoppelt, insbesondere mit einer Bremse, die auf ein an dem Gestell angeordnetes Rad wirkt.

Die Vorrichtung kann so vom Verwender zum Einsatzort geschoben und dort leicht festgesetzt werden.

Bei einer bevorzugten Ausführungsform ist insbesondere vorgesehen, dass der Verwender der Vorrichtung den Griff zum Freigeben der Bremse drücken muss. Die Vorrichtung kann so nur mit gedrücktem Griff verfahren werden und wird automatisch abgebremst, sobald der Benutzer den Griff loslässt.

Bei einer Weiterbildung der Erfindung ist die Auflage längenverstellbar, etwa durch mehrere ineinander verschiebbare Platten, oder kann gegenüber einem Träger horizontal verfahren werden, insbesondere zwischen den vorstehend beschriebenen Seitenteilen mit den Spulen.

Dies ermöglicht eine noch bessere Anpassung an die Anatomie der jeweils zu behandelnden Gliedmaßen.

Die Vorrichtung umfasst vorzugsweise ein Gestell mit einer Breite zwischen 30 und 60 cm und einer Tiefe zwischen 30 und 100 cm.

Die Auflage hat vorzugsweise eine Fläche von 15-40 cm x 20-80 cm und ist so an die Behandlung von Teilbereichen der Gliedmaße, wie z.B. von Gelenken, angepasst.

### Kurzbeschreibung der Zeichnungen

Der Gegenstand der Erfindung soll im Folgenden bezugnehmend auf die Zeichnungen Fig. 1 bis Fig. 6 näher erläutert werden.
Fig. 1 und Fig. 2 sind perspektivische Ansichten eines Ausführungsbeispiels einer erfindungsgemäßen Behandlungsvorrichtung.
Fig. 3 und Fig. 4 sind Detailansichten der Behandlungsvorrichtung.
Fig. 5 und Fig. 6 zeigt ein alternatives Ausführungsbeispiel einer Behandlungsvorrichtung, welches eine zusätzliche Verkleidung aufweist.
Bezugnehmend auf Fig. 7 sollen weitere Details sowie eine leicht geänderte Ausführungsform einer Behandlungsvorrichtung erläutert werden.

### Detaillierte Beschreibung der Zeichnungen

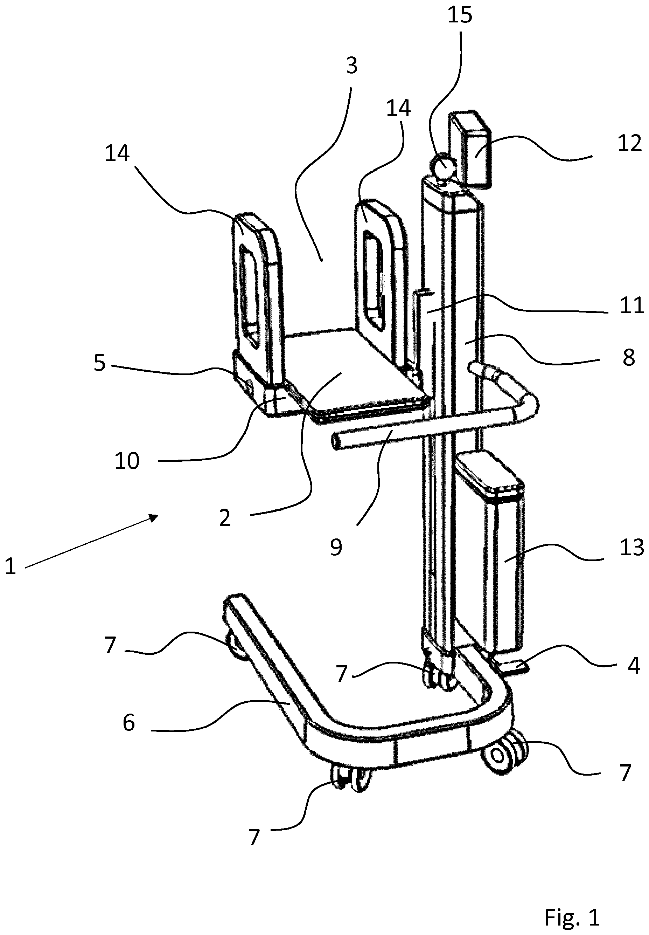

Fig. 1 zeigt ein Ausführungsbeispiel einer mobilen Behandlungsvorrichtung 1 in perspektivischer Ansicht.

Die mobile Behandlungsvorrichtung 1 umfasst eine Auflage 2, die der Positionierung der Gliedmaßen eines Benutzers (nicht dargestellt) dient.

Die Auflage 2 ist derart dimensioniert, dass der Arm oder das Bein eines Benutzers auf der Auflage 2 ruhen kann.

Die mobile Behandlungsvorrichtung 1 umfasst ein U-förmiges Gestell 6, welches Räder 7 aufweist, um die Behandlungsvorrichtung 1 verfahren zu können.

An einer Seite des durch das Gestell 6 gebildeten U erstreckt sich in vertikaler Richtung eine Säule 8 nach oben. Die Säule 8 hat eine Höhe von mindestens 1 m.

Seitlich von der Säule 8 erstreckt sich in Richtung des Gestells über dem Gestell die Auflage 2.

Hierzu ist ein Träger 10 über einen Arm 11 an der Säule 8 angeschlagen.

Der Arm 11 und damit der Träger 10 mit der Auflage ist höhenverstellbar. So kann die Auflage 2 etwa zur Behandlung von Arm oder Bein oder zur Anpassung an die Anatomie des Benutzers hoch- und runtergefahren werden.

Hierzu verfügt dieses Ausführungsbeispiel über einen Hubmechanismus, welcher in der Säule 8 integriert ist und welcher über das Pedal 4 betätigt wird.

Es ist insbesondere vorgesehen, dass über das Pedal 4 die Auflage pneumatisch durch wiederholtes Treten des Pedals 4 nach oben gepumpt wird. Weiter ist vorgesehen, dass die Auflage auch wieder heruntergelassen werden kann, beispielsweise dadurch, dass der Verwender der Behandlungsvorrichtung 1 das Pedal nach oben drückt.

Um die Behandlungsvorrichtung 1 nah an den Benutzer fahren zu können, ist eine Seite des U, an dessen Ende die Säule 8 angebracht ist, kürzer ausgebildet als die gegenüberliegende Seite.

Die Auflage 2 ist des Weiteren noch gegenüber der Horizontalposition im Winkel veränderbar, indem der Träger 10 um die Achse 5 gedreht werden kann. Die Achse 5 verläuft in horizontaler Ebene ausgehend vom Arm 8.

An der Säule 8 ist in diesem Ausführungsbeispiel im unteren Bereich ein Steuergerät 13 angeordnet, welches die Elektrik und Elektronik der Behandlungsvorrichtung sowie ggf. ein Kartenlesegerät (nicht dargestellt) umfasst.

Am oberen Ende der Säule 8 ist ein Bildschirm 12 angeordnet. Dieser ist in diesem Ausführungsbeispiel über ein Gelenk 15 mit der Säule 8 schwenkbar verbunden. Vorzugsweise ist das Gelenk 15 gleichzeitig um eine vertikale Achse drehbar. Der Verwender kann so den Bildschirm 12 immer in seine Richtung drehen.

Vorzugsweise ist der Bildschirm 12 als Touchscreen ausgebildet und dient auch der Bedienung der Behandlungsvorrichtung 1.

Um Kernspinresonanzen in der über der Auflage 2 angeordneten Behandlungszone 3 zu erzielen, erstrecken sich von dem Träger 10 zwei Seitenteile 14 vertikal nach oben. Die Stellung der Seitenteile 14 korrespondiert mit der Stellung der beiden Seitenteile des U, welches durch das Gestell 6 gebildet wird.

Über die Seitenteile 14, in welchen jeweils eine Spule (nicht dargestellt) angeordnet ist, kann ein homogenes Magnetfeld in der Behandlungszone erzielt werden, welches sich über der Auflage 2 von einem Seitenteil 14 zum anderen Seitenteil 14 erstreckt.

Senkrecht zu diesem Magnetfeld kann über eine in oder unter der Auflage angeordnete Spule (nicht dargestellt) ein magnetisches Wechselfeld eingestrahlt werden.

So können Kernspinresonanzen in den Gliedmaßen des Benutzers erzeugt werden.

Die Behandlungsvorrichtung 1 umfasst des Weiteren einen Griff 9, der in diesem Ausführungsbeispiel L-förmig ausgebildet ist und erstreckt sich, von der Säule 8 ausgehend, über die durch das Gestell 6 gebildete Basis des U.

Der Griff 9 ist mit einer Bremse (hier nicht dargestellt) gekoppelt. Diese kann beispielsweise auf ein oder mehrere Räder 7 greifen. Hierzu kann der Griff 9 über einen oder mehrere Seilzüge (nicht dargestellt) mit einer im Rad 7 bzw. den Rädern 7 integrierten Bremse gekoppelt sein. Drückt der Verwender den Griff 9 nach unten, wo wird die Bremse freigegeben und die mobile Behandlungsvorrichtung 1 kann verfahren werden.

Beim Loslassen des Griffs 9 wird die Bremse automatisch angezogen.

Träger 10, Säule 8 und/oder Gestell 6 können aus Metall ausgebildet sein oder Metallteile umfassen. Vorzugsweise sind diese Teile aus Metall elektrisch nicht miteinander verbunden, insbesondere durch Isolierelemente aus Kunststoff (nicht dargestellt). So wird die Bildung großflächiger Antennen, welche störende Magnetfelder erzeugen können, vermieden.

Die Auflage 2 ist in diesem Ausführungsbeispiel als auf dem Träger 10 angeordneter Schlitten ausgebildet, welcher zwischen den Seitenteilen 14 horizontal verfahren werden kann.

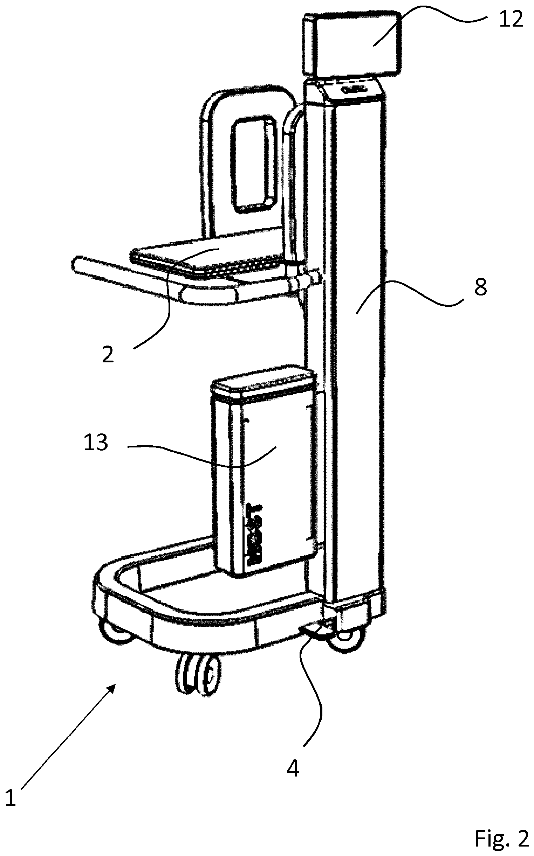

Fig. 2 ist eine weitere perspektivische Ansicht der in Fig. 1 dargestellten Behandlungsvorrichtung 1.

Gut zu erkennen ist die Säule 8, an deren unteren Ende das Pedal 4 angeordnet ist, mittels dessen die Höhe der Auflage 2 verändert werden kann.

Weiter zu erkennen ist das Steuergerät 13, welches die elektrischen und elektronischen Komponenten zur Ansteuerung der Spulen in den Seitenteilen 14 sowie unter der Auflage aufweist.

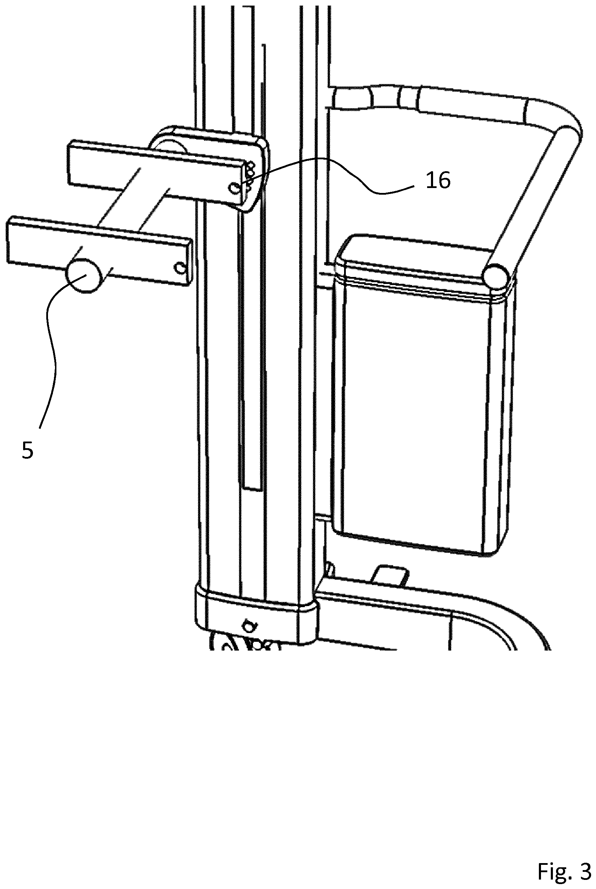

Fig. 3 ist eine Detailansicht, in welcher der Träger nebst Auflage ausgeblendet ist.

Zu erkennen ist die Achse 5, über die der Winkel der Auflage gegenüber der Horizontalen verändert werden kann.

Hierzu umfasst die Vorrichtung einen Rastmechanismus 16. Dieser kann einen hier nicht dargestellten Stift umfassen, der in die zu erkennenden Löcher greift.

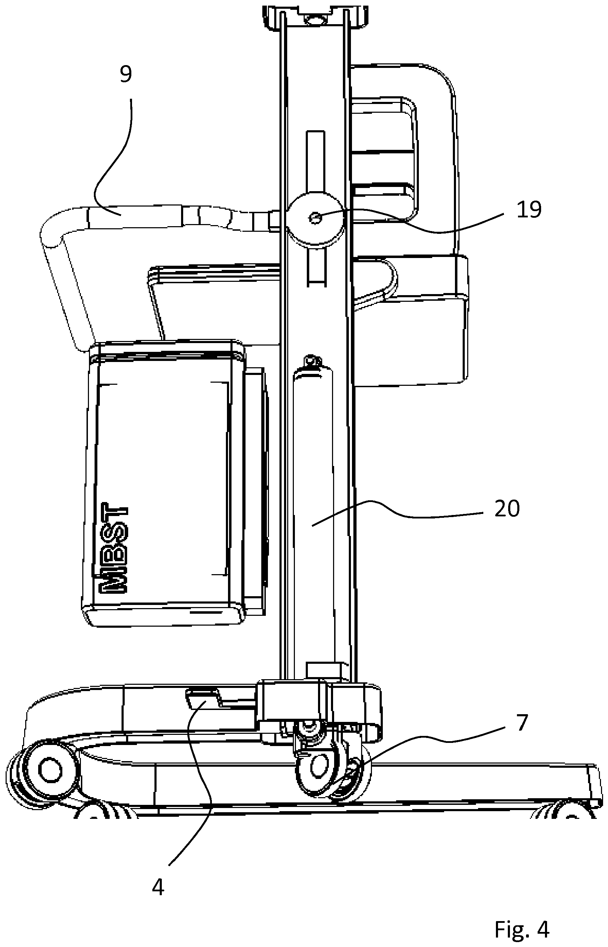

Fig. 4 ist eine weitere Detailansicht, in welcher das äußere Gehäuse der Säule ausgeblendet ist.

Zu erkennen ist, dass der Griff 9 über ein Gelenk 19 mit der Säule 8 verbunden ist. So kann der Griff 9 herabgedrückt werden, um die Bremse freizugeben, welche vorzugsweise auf ein Rad 7 greift.

Weiter zu erkennen ist der Pneumatikzylinder 20, welcher Teil der über das Pedal 4 betätigbaren Hubvorrichtung ist.

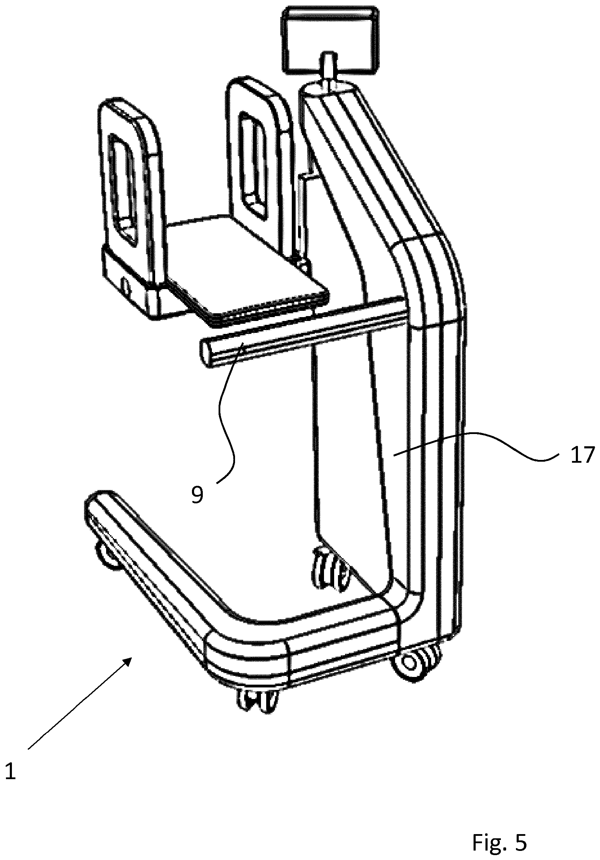

Fig. 5 ist ein weiteres Ausführungsbeispiel einer mobilen Behandlungsvorrichtung 1, welches im Wesentlichen der in den vorherigen Figuren gezeigten Behandlungsvorrichtung entspricht mit dem Unterschied, dass eine Verkleidung 17 angebracht ist, die sich um das U-förmige Gestell und die Säule 8 erstreckt.

Es ragt nunmehr nur noch das Ende des Griffs 9 aus der Verkleidung 17.

Die Funktionalität der Behandlungsvorrichtung ist ansonsten identisch.

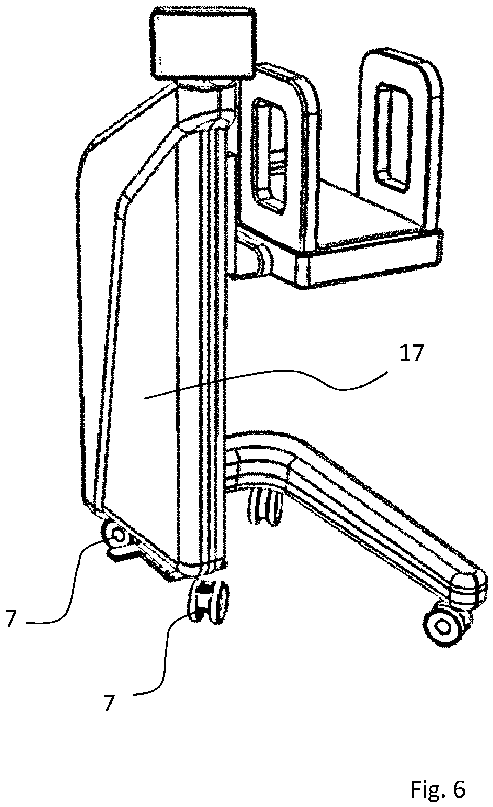

Fig. 6 ist eine weitere perspektivische Ansicht der mit der Verkleidung 17 versehenen Behandlungsvorrichtung 1.

Zu sehen ist, dass sich die Verkleidung 17 auf der kürzeren Seite des U über den gesamten Bereich zwischen den Rädern 7 erstreckt.

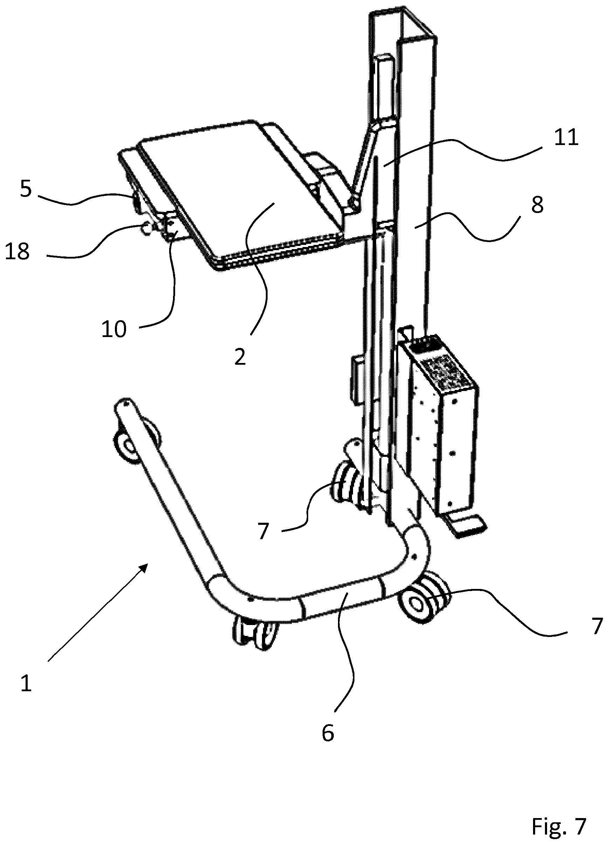

Bezugnehmend auf Fig. 7 sollen weitere Details sowie eine weitere, leicht abgewandelte Ausführungsvariante einer mobilen Behandlungsvorrichtung 1 erläutert werden.

Im Unterschied zu den zuvor dargestellten Ausführungsvarianten ist die Säule 8 nicht am Ende der kürzeren Seite des durch das Gestell 6 gebildeten U angeordnet, sondern in Richtung der Unterseite des U versetzt.

Es hat sich gezeigt, dass hierdurch eine noch bessere Zugänglichkeit der Gliedmaßen des Benutzers ermöglicht wird.

Ansonsten entspricht die Behandlungsvorrichtung 1 der in Fig. 1 und 2 dargestellten Behandlungsvorrichtung.

Zu erkennen ist hier, da nunmehr die Seitenteile ausgeblendet sind, der Arm 17, welcher höhenverstellbar an der Säule 8 angeordnet ist.

Der Arm 11 ist L-förmig ausgebildet.

Weiter zu erkennen ist der Träger 10 für die Auflage 2.

Aus dem Träger 10 ragt ein Betätigungsorgan 10 für den Rastmechanismus hervor.

Um den Winkel der Auflage 2 gegenüber der Horizontalen zu variieren, kann der Verwender der Behandlungsvorrichtung 1 das Betätigungsorgan 18, etwa durch Ziehen oder Drücken, betätigen. Der Rastmechanismus wird freigegeben und die Auflage 2 kann über die Achse 5 verschwenkt werden.

In dem gewünschten Winkel kann sodann die Auflage 2 wieder verrastet werden.

Durch die Erfindung konnte eine mobile Behandlungsvorrichtung bereitgestellt werden, welche sich auf sehr einfache Weise komfortabel den anatomischen Gegebenheiten zur Behandlung der Gliedmaßen eines Benutzers anpassen lässt.

### Bezugszeichenliste

- 1: mobile Behandlungsvorrichtung
- 2: Auflage
- 3: Behandlungszone
- 4: Pedal
- 5: Achse
- 6: Gestell
- 7: Rad
- 8: Säule
- 9: Griff
- 10: Träger
- 11: Arm
- 12: Bildschirm
- 13: Steuergerät
- 14: Seitenteil
- 15: Gelenk
- 16: Rastmechanismus
- 17: Verkleidung
- 18: Betätigungsorgan
- 19: Gelenk
- 20: Pneumatikzylinder

## Patentansprüche

1. Mobile Behandlungsvorrichtung (1) mit einer Auflage (2) für die Gliedmaße eines Benutzers, wobei in einem über der Auflage (2) angeordneten Behandlungsvolumen Kernspinresonanzen im Gewebe des Benutzers erzeugbar sind, und wobei die Auflage (2) höhenverstellbar ist wobei die mobile Behandlungsvorrichtung (1) ein Gestell (6) aufweist, von welchem ausgehend sich eine Säule (8) vertikal erstreckt, wobei die Auflage (2) an der Säule (8) und über dem Gestell (6) angeordnet ist,
wobei das Gestell Räder (7) aufweist, um die Behandlungsvorrichtung verfahren zu können, **dadurch gekennzeichnet, dass** das Gestell U-förmig ausgebildet ist,
wobei an einer Seite des durch das Gestell gebildeten U die Säule (8) mit der Auflage (2) angeordnet ist, wobei diese Seite des U kürzer als die gegenüberliegende Seite des U ausgebildet ist.

2. Mobile Behandlungsvorrichtung (1) nach dem vorstehenden Anspruch, wobei die Auflage (2) über einen Hubmechanismus höhenverstellbar ist, insbesondere einen Hubmechanismus, welcher mittels eines Pedals (4) betätigbar ist.

3. Mobile Behandlungsvorrichtung (1) nach einem der vorstehenden Ansprüche, wobei die Auflage (2) in einer horizontal verlaufenden Achse (5) drehbar ausgebildet ist.

4. Mobile Behandlungsvorrichtung (1) nach dem vorstehenden Anspruch, wobei die Hubmechanik in der Säule (8) integriert ist.

5. Mobile Behandlungsvorrichtung (1) nach einem der vorstehenden Ansprüche, wobei die mobile Behandlungsvorrichtung (1) einen Griff (9) zum Schieben der Behandlungsvorrichtung (1) aufweist.

6. Mobile Behandlungsvorrichtung nach dem vorstehenden Anspruch, wobei der Griff (9) mit einer Bremse, insbesondere für ein Rad (7), gekoppelt ist.

7. Mobile Behandlungsvorrichtung (1) nach einem der vorstehenden Ansprüche, wobei die Auflage (2) längenverstellbar und/oder gegenüber einem Träger (10) verfahrbar ist.

## Claims

1. A mobile treatment device (1), comprising a support (2) for a user's limb, wherein nuclear magnetic resonances can be generated in the user's tissue in a treatment volume located above the support (2), and wherein the support (2) is adjustable in height;
the mobile treatment device (1) comprising a frame (6) from which a column (8) extends vertically, and wherein the support (2) is arranged on said column (8) and above said frame (6);
wherein the frame has wheels (7) to allow the treatment device to be displaced; **characterized in that** the frame is U-shaped;
wherein the column (8) with the support (2) is arranged on one side of the U defined by the frame, said one side of the U being shorter than the opposite side of the U.

2. The mobile treatment device (1) according to the preceding claim, wherein the support (2) is adjustable in height by a lifting mechanism, in particular a lifting mechanism that can be actuated by a pedal (4).

3. The mobile treatment device (1) according to any one of the preceding claims, wherein the support (2) is configured to be rotatable on a horizontally extending axle (5).

4. The mobile treatment device (1) according to the preceding claim, wherein the lifting mechanism is integrated in the column (8).

5. The mobile treatment device (1) according to any one of the preceding claims, wherein the mobile treatment device (1) comprises a handle (9) for displacing the treatment device (1).

6. The mobile treatment device according to the preceding claim, wherein the handle (9) is coupled to a brake, in particular for a wheel (7).

7. The mobile treatment device (1) according to any one of the preceding claims, wherein the support (2) is adjustable in length and/or displaceable relative to a supporting mount (10).

## Revendications

1. Dispositif de traitement mobile (1) comprenant une surface d'appui (2) pour les membres d'un utilisateur, des résonances magnétiques nucléaires pouvant être générées dans un volume de traitement disposé au-dessus de la surface d'appui (2), dans les tissus de l'utilisateur, et la surface d'appui étant réglable en hauteur, le dispositif de traitement mobile (1) présentant un cadre (6) à partir duquel une colonne (8) s'étend dans le sens vertical, la surface d'appui (2) étant disposée sur la colonne (8) et au-dessus du cadre (6),
le cadre présentant des roues (7) permettant de déplacer le dispositif de traitement, **caractérisé en ce que** le cadre est réalisé en forme de U, la colonne (8), avec la surface d'appui (2), étant disposée sur un côté du U formé par le cadre, ledit côté du U étant plus court que le côté opposé du U.

2. Dispositif de traitement mobile (1) selon la revendication précédente, dans lequel la surface d'appui (2) peut être réglée en hauteur par l'intermédiaire d'un mécanisme de levage, en particulier un mécanisme de levage pouvant être actionné à l'aide d'une pédale (4).

3. Dispositif de traitement mobile (1) selon l'une des revendications précédentes, dans lequel la surface d'appui (2) est réalisée de manière à pouvoir tourner dans un axe (5) horizontal.

4. Dispositif de traitement mobile (1) selon la revendication précédente, dans lequel le mécanisme de levage est intégré dans la colonne (8).

5. Dispositif de traitement mobile (1) selon l'une des revendications précédentes, dans lequel le dispositif de traitement mobile (1) présente une poignée (9) destinée à pousser le dispositif de traitement (1).

6. Dispositif de traitement mobile selon la revendication précédente, dans lequel la poignée (9) est couplée à un frein, en particulier pour une roue (7).

7. Dispositif de traitement mobile (1) selon l'une des revendications précédentes, dans lequel la surface d'appui (2) peut être réglée en longueur et/ou peut être déplacée en translation par rapport à un support (10).
